# EUROPEAN PATENT APPLICATION

(11) **EP 3 262 953 A1**
(43) Date of publication of application: **03.01.2018**
(21) Application number: 17184380.8
(22) Date of filing: 14.12.2012
(51) Int. Cl.: A23K 10/14, A23K 20/147, A23K 20/163, A23K 20/189, A23K 50/48

(54) **METHOD OF PREPARING A FOOD COMPOSITION**

(62) Divisional of application: 12809946.2
(71) Applicant: Colgate-Palmolive Company, New York, NY 10022 (US)
(72) Inventor: KAPPELMAN, David, Olathe, KS Kansas 66062 (US); LINDECRANTZ, Jason, Topeka, KS Kansas 66605 (US)
(74) Representative: Wichmann, Hendrik

(57) **Abstract**

The invention provides a method of preparing a moist animal food composition comprising a food component, wherein the method comprises: treating the food component with transglutaminase and alginate. The invention further provides a method of increasing the firmness of a food component comprising treating the food component with transglutaminase and alginate.

## Description

### BACKGROUND OF THE INVENTION

Enzymes are often incorporated into food compositions to modify properties such as preservability, texture and taste.

Transglutaminase is an enzyme that catalyzes the formation of a covalent bond between a free amine group (e.g., protein- or peptide-bound lysine) and the gamma-carboxamide group of protein- or peptide-bound glutamine. Bonds formed by transglutaminase exhibit high resistance to proteolytic degradation.

Animal food compositions may be classified as "moist" or "dry".

"Moist" food compositions are generally packaged in can-like containers and are considered 'moist' in appearance because of the moisture contained therein. Two types of moist food compositions are generally known in the art.

The first is known in the art as "ground loaf." Loaf products are typically prepared by contacting a mixture of components under heat to produce an essentially homogeneous, intracellular honeycomb-type mass or "ground loaf." The ground loaf mass is then packaged into a cylindrical container, such as a can. Upon packing, ground loaf assumes the shape of the container such that the ground loaf must be cut when serving to a companion animal. As a result of processing, ground loaf products exhibit a wide range of textural differences.

Another type of moist food composition is generally known in the art as "chunk and gravy." Chunk and gravy products comprise pre-formed meat particles that are prepared by making a meat emulsion which is then extruded and formed by physical pressure or thermal energy (for example, by cooking with steam, cooking in water, and oven dry heat). The cooked meat product is diced into chunks, which are subsequently mixed with a gravy or sauce. The two components are then filled into a container, usually a can, which is steamed and sterilized. As opposed to the ground loaf, chunk and gravy compositions comprise physically separate, discrete chunks (i.e., pieces of ground meat and grains). These discrete pieces are present in the gravy-type liquid in the final container.

In order to prepare food compositions, and in particular moist pet food compositions that are nutritionally balanced, it is known to blend various types of meat with cereal grains, vitamins, minerals and other micronutrients. However, such blended compositions may not have the desired structure, or sufficient firmness, for further processing (such as extrusion and dicing). Accordingly, such compositions may require additional treatments such as heating or the formation of a hydrocolloid gel in order to create a suitable structure and firmness for further processing.

It would therefore be desirable to provide improved methods of preparing food compositions with a desirable structure and firmness.

### SUMMARY OF THE INVENTION

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

The present inventors have surprisingly found that the firmness of a food component may be increased, advantageously without requiring added heat or pressure, or hydrocolloid gel formation, by treating the food component with transglutaminase and at least one further agent selected from gluten and alginate.

Accordingly, in a first aspect, the present invention provides a method of preparing a moist animal food composition comprising a food component, wherein the method comprises:
treating the food component with transglutaminase and at least one further agent selected from gluten and alginate,
and firming the food component in the absence of added heat or pressure.

Typically, the treating and firming are conducted simultaneously.

Optionally, the at least one further agent selected is gluten. Alternatively, the at least one further agent selected is alginate.

Typically, the firming step is conducted at a temperature of from 3°C to 23°C. Optionally, the firming step is conducted at a temperature of from 4°C to 10°C. Further optionally, the firming step is free of a heating step. Still further optionally, the firming is conducted at atmospheric pressure (which is typically about 101 kPa).

In the method of the present invention, the food component is optionally treated with transglutaminase and the at least one further agent selected from gluten and alginate, for three to seventy-two hours, or for six to eighteen hours.

Further optionally, the firming is carried out in the absence of exogenous moisture.

Typically, the food component comprises a protein source. Optionally, the protein source is an animal protein source. The protein source may comprise both an animal protein source and a vegetable protein source. Optionally, the protein source comprises meat, a meat by-product or fish. Further optionally, the protein source comprises chicken, beef, pork or turkey. Preferably, the protein source comprises beef or chicken.

Typically, the food component is combined with one or more food ingredients. Optionally, the food component is combined with one or more food ingredients before treating and/or firming. The food ingredients may be selected from grains, cereal flour, starch, fats, oils, vitamins, minerals, colorants, flavorants, amino acids, fiber, and inorganic additives.

Optionally, the food component further comprises one or more food ingredients that are selected from grains, cereal flour, starch, fats, oils, vitamins, minerals, colorants, flavorants, amino acids, fiber, and inorganic additives.

Optionally, the food component is treated with transglutaminase in an amount of 1 unit of enzyme activity/g to 4 units of enzyme activity/g of the food component. Further optionally, the food component is treated with transglutaminase in an amount of 1 unit of enzyme activity/g to 3 units of enzyme activity/g of the food component. Still further optionally, the food component is treated with transglutaminase in an amount of 1 unit of enzyme activity/g to 2 units of enzyme activity/g of the food component.

Optionally, the food component is treated with gluten in an amount of 0.05 wt% to 10 wt% by total weight of the food component. Further optionally, the food component is treated with gluten in an amount of 0.5 wt% to 5 wt%. Still further optionally, the food component is treated with gluten in an amount of 0.5 wt% to 3 wt% by total weight of the food component.

Optionally, the food component is treated with alginate in an amount of 0.01 wt% to 3 wt% by total weight of the food component. Further optionally, the food component is treated with alginate in an amount of 0.1 wt% to 1.5 wt% by total weight of the food component.

Typically, the alginate comprises sodium alginate, potassium alginate, ammonium alginate, calcium alginate and propylene glycol alginate.

In the method according to the present invention, the animal food composition may be extruded and optionally, cut. The food component may also be combined with one or more food ingredients selected from meat, grain, cereal flour, starch, fats, oils, vitamins, minerals, colorants, flavorants, amino acids, inorganic additives and water.

Optionally, the animal food composition is cured, and further optionally, overwrapped. Still further optionally, the animal food composition is vacuum-sealed.

Typically, the animal is a companion animal.

In a second aspect, the present invention provides a method of increasing the firmness of a food component comprising:
treating the food component with transglutaminase and at least one further agent selected from gluten and alginate, in the absence of added heat or pressure.

Optionally, the at least one further agent selected is gluten. Alternatively, the at least one further agent selected is alginate.

Typically, the food component is treated with transglutaminase and the at least one further agent selected from gluten and alginate, at a temperature of from 3°C to 23°C. Optionally, the temperature is from 4°C to 10°C. Further optionally, the treatment is free of a heating step. Still further optionally, the treatment is conducted at atmospheric pressure (which is typically about 101 kPa).

In the second aspect, the food component is optionally treated with transglutaminase and the at least one further agent selected from gluten and alginate, for six to seventy-two hours, or for twelve to eighteen hours.

Further optionally, the method is carried out in the absence of exogenous moisture.

Typically, the protein source comprises a protein source. Optionally, the protein source is an animal protein source. The protein source may comprise both an animal protein source and a vegetable protein source. Optionally, the protein source comprises meat, a meat by-product or fish. Further optionally, the protein source comprises chicken, beef, pork or turkey. Preferably, the protein source comprises beef or chicken.

Typically, the food component is combined with one or more food ingredients. Optionally, the food component is combined with one or more food ingredients before treating and/or firming. The food ingredients may be selected from grains, cereal flour, starch, fats, oils, vitamins, minerals, colorants, flavorants, amino acids, fiber, and inorganic additives.

Typically, the food component further comprises one or more food ingredients that are selected from grains, cereal flour, starch, fats, oils, vitamins, minerals, colorants, flavorants, amino acids, fiber, and inorganic additives.

Optionally, the food component is treated with transglutaminase in an amount of 1 unit of enzyme activity/g to 4 units of enzyme activity/g of the food component. Further optionally, the food component is treated with transglutaminase in an amount of 1 unit of enzyme activity/g to 3 units of enzyme activity/g of the food component. Still further optionally, the food component is treated with transglutaminase in an amount of 1 unit of enzyme activity/g to 2 units of enzyme activity/g of the food component.

Optionally, the food component is treated with gluten in an amount of 0.05 wt% to 10 wt% by total weight of the food component. Further optionally, the food component is treated with gluten in an amount of 0.5 wt% to 5 wt%. Still further optionally, the food component is treated with gluten in an amount of 0.5 wt% to 3 wt% by total weight of the food component.

Optionally, the food component is treated with alginate in an amount of 0.01 wt% to 3 wt% by total weight of the food component. Further optionally, the food component is treated with alginate in an amount of 0.1 wt% to 1.5 wt% by total weight of the food component.

Typically, the alginate comprises sodium alginate, potassium alginate, ammonium alginate, calcium alginate and propylene glycol alginate.

In a third aspect, the present invention provides an animal food composition comprising:
a protein source,
transglutaminase in an amount of 1 unit of enzyme activity/g to 4 units of enzyme activity/g of the food component,
   and
at least one agent selected from gluten in an amount of 0.05 wt% to 10 wt% by total weight of the food component, and alginate in an amount of 0.01 wt% to 3 wt% by total weight of the food component.

Optionally, the food composition comprises transglutaminase in an amount of 1 unit of enzyme activity/g to 3 units of enzyme activity/g of the food component. Further optionally, the food component comprises transglutaminase in an amount of 1 unit of enzyme activity/g to 2 units of enzyme activity/g of the food component.

Optionally, the food composition comprises alginate in an amount of 0.1 wt% to 1.5 wt% by total weight of the food composition. The alginate may comprise sodium alginate, potassium alginate, ammonium alginate, calcium alginate and propylene glycol alginate. Optionally, the food composition comprises gluten in an amount of 0.5 wt% to 10 wt% by total weight of the food composition.

Typically, the protein source comprises an animal protein source. The protein source may comprise both an animal protein source and a vegetable protein source. Optionally, the protein source comprises meat, a meat by-product or fish. Further optionally, the protein source comprises chicken, beef, pork or turkey. Preferably, the protein source comprises beef or chicken.

Typically, the food composition further comprises one or more food ingredients that are selected from grains, cereal flour, starch, fats, oils vitamins, minerals, colorants, flavorants, amino acids, fiber, and inorganic additives.

Typically, the animal is a companion animal.

Optionally, the food composition comprises a meat analogue. Further optionally, the food composition is cured and optionally, overwrapped. Still further optionally, the animal food composition is vacuum-sealed.

In a fourth aspect, the present invention provides a use of transglutaminase and gluten to increase the firmness of a food component in the absence of added heat or pressure.

In a fifth aspect, the present invention provides a use of transglutaminase and alginate to increase the firmness of a food component in the absence of added heat or pressure.

The methods and uses of the present invention provide a new means by which the firmness of a food component may be increased, without requiring heat or pressure, or hydrocolloid gel formation. The present inventors have surprisingly found that the combination of transglutaminase and gluten, or the combination of transglutaminase and alginate, provides a synergistic effect on the structuring and firming of protein-containing food components.

### DETAILED DESCRIPTION

The following description of the preferred embodiment(s) is merely exemplary in nature and is in no way intended to limit the invention, its application, or uses.

In some embodiments, the present invention provides a method of preparing an animal food composition comprising a food component, wherein the method comprises: treating the food component with transglutaminase and at least one further agent selected from gluten and alginate,
and firming the food component in the absence of added heat or pressure.

### Animal

An animal in the context of the present invention may be any mammal or bird. The animal food composition of the present invention may accordingly be formulated for any mammal or bird. Preferably, the animal food composition of the present invention is formulated for a companion animal. Companion animals include canines, felines, ferrets and rodents. In one arrangement, the food composition according to the invention is for a canine or a feline.

### Food component

The food component may comprise a protein source. The protein source of the food component may comprise an animal protein source and/or a vegetable protein source. Preferably, the protein source comprises an animal protein source. More preferably, the animal protein source is selected from meat, meat by-products and fish. Meat sources may comprise animal muscle, animal skeletal meat, animal by-products, and mixtures of muscle, skeletal meat and byproducts. Meats include, for example, the flesh of poultry and mammals (e.g., chickens, cattle, swine, sheep, goats, and the like). Meat by-products include, for example, lungs, kidneys, livers, tongues, stomachs and intestines. Suitable animal protein sources may include fresh and frozen meats or meat by-products. In a preferred embodiment, the meat source comprises beef or chicken.

Vegetable protein sources suitable for preparing compositions of the invention include, but are not limited to, potato concentrate, soy concentrate, soy protein isolate, soybean meal, corn gluten meal, rice protein isolate, pea protein concentrate, wheat protein concentrate, and wheat protein isolate. Vegetable protein may be isolated from any portion of a plant, isolated from more than one portion of a plant, and isolated from more than one plant by methods known by those of skill in the art. Vegetable protein may also be concentrated by methods known by those of skill in the art.

In the method of the present invention, the food component may additionally be combined with one or more food ingredients that are selected from grains, cereal flour, starch, fats, oils, vitamins, minerals, colorants, flavorants, amino acids, fiber and inorganic additives to form the food composition. In one embodiment, the food component is combined with one or more food ingredients before treating and/or firming. In another embodiment, the food component is combined with one or more food ingredients after treating and/or firming.

Alternatively or additionally, the food component may comprise one or more food ingredients that are selected from grains, cereal flour, starch, fats, oils, vitamins, minerals, colorants, flavorants, amino acids, fiber and inorganic additives.

Suitable grains include, for example, grains selected from the group consisting of oat fiber, cellulose, peanut hull, beet pulp, parboiled rice, corn starch, corn gluten meal and mixtures thereof. It is important to note that by properly balancing carbohydrate sources, one skilled in the art can manipulate the texture of the final product. For example, short chain polysaccharides tend to be more 'sticky' and 'gluey' than longer chain polysaccharides.

The amount of the optional additives combined with or comprised in the food component is at least partially dependent on the nutritional requirements for animals at different stages of life. The Association of American Feed Control Officials (AAFCO), for example, provides recommended amounts of such ingredients for dogs and cats. (See Association of American Feed Control Officials, Inc., Official publication, pp. 147-153 (2011)).

Vitamins generally useful as food additives include, for example, vitamin A, vitamin B1, vitamin B2, vitamin B6, vitamin B 12, vitamin C, vitamin D, vitamin E, vitamin H (biotin), vitamin K, folic acid, inositol, niacin, and pantothenic acid. Minerals and trace elements generally useful as food additives include, for example, calcium, phosphorus, sodium, potassium, magnesium, copper, zinc, choline, and iron salts.

Fat can be supplied by any of a variety of sources known by those skilled in the art, including meat, meat by-products, fish oil, and plants. Plant fat sources include wheat, flaxseed, rye, barley, rice, sorghum, corn, oats, millet, wheat germ, corn germ, soybeans, peanuts, and cottonseed, as well as oils derived from these and other plant fat sources.

Amino acids, including essential amino acids, free amino acids, or amino acids supplied by any number of sources, e.g., crude protein, may be present in the food component of the present invention. Essential amino acids are amino acids that cannot be synthesized de novo, or in sufficient quantities by an organism and thus must be supplied in the diet. Essential amino acids vary from species to species, depending upon the organism's metabolism. For example, it is generally understood that the essential amino acids for dogs and cats (and humans) are phenylalanine, leucine, methionine, lysine, isoleucine, valine, threonine, tryptophan, histidine and arginine. In addition, taurine, which is technically not an amino acid but a derivative of cysteine, is an essential nutrient for cats.

The food component may additionally comprise, or be combined with, a source of dietary fiber. Dietary fiber refers to components of a plant which are resistant to digestion by an animal's digestive enzymes. Dietary fiber includes soluble and insoluble fibers. Soluble fibers are resistant to digestion and absorption in the small intestine and undergo complete or partial fermentation in the large intestine. Soluble fibers include beet pulp, guar gum, chicory root, psyllium, pectin, blueberry, cranberry, squash, apples, oats, beans, citrus, barley, or peas. Insoluble fibers may be supplied by any of a variety of sources, including cellulose, whole wheat products, wheat oat, corn bran, flax seed, grapes, celery, green beans, cauliflower, potato skins, fruit skins, vegetable skins, peanut hulls, and soy fiber.

The food component may additionally comprise, or be combined with stabilizers, fillers, thickeners, and palatability enhancers.

In one embodiment, the food component is a meat mixture is prepared by mixing meat and/or meat by-products with one or more of the optional food ingredients defined above. The prepared meat mixture or meat component for inclusion in the animal food composition generally comprises at least 15% by weight protein and at least 5% by weight fat. For example, in one embodiment, the meat mixture comprises from 15% to 50% by weight protein, and from 5% to 30% by weight fat.

In some embodiments, the food component or food composition of the present invention comprises a meat analogue. The term "meat analogue" denotes textured protein resembling meat which may be cut into pieces, having the appearance of whole meat pieces. Meat analogues may comprise vegetable protein sources such as soybean, wheat, cottonseed, sunflower seed, and corn. Other protein sources such as fish meal, keratin, algae and kelp may also be included in the meat analogues herein. Additionally, meat analogues may further comprise meat or meat by-products, including chicken, turkey, beef, and pork. The meat analogues may still further comprise meat flavors, spices, fat, synthetic fat, animal tissue and other materials which make them more "meat-like."

### Treating and Firming

By "treating" it is meant that the food component is brought into contact with the transglutaminase and the at least one further agent selected from gluten and alginate ("the active agents").

The food component may be treated with the active agents under conditions in which firming of the food component occurs. Accordingly, the treating step may include the firming of the food component, and treating and firming may occur simultaneously. Therefore, the conditions of firming defined herein may also be the conditions under which the food component is treated. Similarly, the conditions under which the food component is treated as defined herein may also be the conditions of firming.

In another embodiment, the food component may be treated with the active agents, and subsequently firmed. In such an embodiment, the treating and firming may occur in succession.

In some embodiments of the present invention, the firmness of the food component is increased by treating the food component with transglutaminase and at least one further agent selected from gluten and alginate.

The active agents may comprise transglutaminase and gluten, transglutaminase and alginate, or transglutaminase, alginate and gluten. The food component may be treated with the active agents in a simultaneous or sequential manner.

Thus for example, in one arrangement, a meat mixture is prepared by simultaneously mixing meat and/or meat by-products with one or more optional food ingredients defined above, transglutaminase, and at least one agent selected from gluten and alginate. Such a meat mixture may be prepared in any suitable mixing apparatus known to the person skilled in the art. Non-limiting examples of suitable apparatus for preparing the meat mixture include a twin screw mixer, a twin ribbon mixer, an overlapping paddle mixer, or a combination mixer such as a screw/ribbon/paddle.

In an alternative arrangement, a meat mixture may be prepared by mixing meat and/or meat by-products with one or more optional food ingredients defined above and with transglutaminase, in the absence of any further active agents. Gluten and/or alginate may subsequently be incorporated into the mixture.

Transglutaminase may be obtained from several plant and animal sources, as would be known to the person skilled in the art. Transglutaminase preparations are also available commercially.

In one arrangement, the food component is treated with transglutaminase in an amount of 1 unit of enzyme activity/g to 4 units of enzyme activity/g of the food component, or in an amount of 1.5, 2, or 2.5 units of enzyme activity/g to 3, 3.5 or 4 units of enzyme activity/g of the food component. Optionally, the food component is treated with transglutaminase in an amount of 1 unit of enzyme activity/ g to 3 units of enzyme activity/g of the food component, or in an amount of 1.5 or 2 units of enzyme activity/g to 2.5 or 3 units of enzyme activity/g of the food component. Still further optionally, the food component is treated with transglutaminase in an amount of 1 unit of enzyme activity/g to 2 units of enzyme activity/g of the food component, or in an amount of 1.1, 1.2, 1.3, or 1.4 units of enzyme activity/g, to 1.5, 1.6, 1.7, 1.8 or 1.9 units of enzyme activity/g of the food component.

The alginate may be in the form of sodium alginate, potassium alginate, ammonium alginate, calcium alginate and propylene glycol alginate. In one arrangement, the food component is treated with alginate in an amount of 0.01 wt% to 3 wt% by total weight of the food component, or in an amount of from 0.05 wt% to 2 wt% by total weight of the food component. In one arrangement, the food component is treated with alginate in an amount of from 0.1 wt%, or 0.2 wt%, or 0.3 wt% or 0.4 wt% or 0.5 wt% to 1 wt%, or 1.5 wt% or 2 wt% by total weight of the food component. Preferably, the food component is treated with alginate in an amount of 0.1 wt% to 1.5 wt% by total weight of the food component, or in an amount of 0.5 wt% to 1 wt% by total weight of the food component.

Gluten may be obtained from wheat and related grain species including barley, rye and corn. In one arrangement, the food component is treated with gluten in an amount of 0.05 wt% to 10 wt% by total weight of the food component, or in an amount of from 0.1 wt% to 5 wt% by total weight of the food component, or in an amount of from 0.1 wt% to 5 wt% by total weight of the food component. Preferably, the food component is treated with gluten in an amount of 0.5 wt% to 3 wt% by total weight of the food component. In one arrangement, the food component is treated with gluten in an amount of from 0.6 wt%, or 0.7 wt%, or 0.8 wt% or 0.9 wt% or 1 wt% to 1 wt%, or 1.5 wt%, or 2 wt%, or 2.5 wt%, 3 wt%, 5 wt%, 7 wt% or 10 wt% by total weight of the food component. In another arrangement, the food component is treated with gluten in an amount of 1 wt% to 1.5 wt% by total weight of the food component.

Typically, firming is conducted at a temperature of from 3°C to 23°C. In a preferred embodiment, firming is conducted at a temperature of from 4°C to 10°C, or from 4°C to 8°C, or from 4°C to 6°C. The present inventors have surprisingly found that transglutaminase is sufficiently active at these temperatures to promote firming of the food component, in the presence of gluten or alginate. Therefore, advantageously, no further heating step is required to achieve firming of the food component. Accordingly, in one arrangement, the food component is treated or firmed with transglutaminase and the at least one further agent selected from gluten and alginate in the absence of heating. Thus, the firming may be free, or substantially free of a heating step. Preferably, the temperature is not raised above room temperature (which is typically about 23°C).

In some embodiments, the food component may be treated with the active agents at the temperatures defined herein so as to achieve firming. In such an arrangement, the treating and firming are conducted simultaneously. Accordingly, the food component may be treated and/or firmed at the temperatures defined herein.

Typically, the food component is treated with transglutaminase and the at least one agent selected from gluten and alginate over a period of three to seventy two hours. In a preferred arrangement, the treatment occurs over a period of from six to twenty four hours, or from six to eighteen hours, or from six to twelve hours.

In some embodiments, the food component may be treated with the active agents at for the times defined herein so as to achieve firming (i.e., such that treating and firming are conducted simultaneously). Accordingly, the treatment and/or firming may be conducted over a period of from three to seventy two hours, from six to twenty four hours, from six to eighteen hours, or from six to twelve hours.

The present inventors have surprisingly found that firming may be achieved in the absence of added pressure, in addition to the absence of added heat. Thus, typically, the treatment and/or firming is conducted at atmospheric pressure (which is typically 101 kPa), in the absence of any added pressure.

In one arrangement, the firming is carried out in the absence of exogenous moisture. In one embodiment, particularly wherein the treatment encompasses firming, the treatment may also be carried out in the absence of exogenous moisture.

### Further processing

In some embodiments of the invention, the food component which has been subjected to firming (the "firmed" food component) is optionally combined with one or more food ingredients. The optional combination with food ingredients may take place prior to, or after, further processing of the food component. Such food ingredients include, without limitation, grains, cereal flour, starch, fats, oils, vitamins, minerals, colorants, flavorants, amino acids, fiber, stabilizers, fillers, thickeners, palatability enhancers and inorganic additives as described above.

Further processing may include extrusion, cutting, and canning, or any other method or process of producing animal foods that is known in the art.

Typically, the food compositions of the present invention are prepared in a canned or moist form using conventional food preparation processes known to the person skilled in the art. "Moist food" refers to a food composition that has a moisture content of about 60 to 90% or greater.

In one arrangement, the firmed food component and optional food ingredients are extruded and cut into pieces. The extruded pieces may be transferred into cans with a gravy sauce, sealed and sterilized.

In an alternative arrangement, the extruded pieces are combined with ground meats, cereal grains and any of the optional food ingredients defined herein, prior to canning and sterilization. By this method, a "chunks in loaf" style animal food composition may be prepared. Other optional food ingredients include vegetables, grains, potato or sweet potato.

In yet another alternative arrangement, the extruded pieces are combined with ground meats, cereal grains and any of the optional food ingredients defined herein, overwrapped in a casing, and cured at about 3°C. The cured food composition may subsequently be sold and stored under refrigerated conditions, and heated for animal consumption.

In still yet another alternative arrangement, the extruded pieces may be vacuum sealed alone, or in combination with other ingredients. Other ingredients may include gravy or sauce, vegetables, potato, sweet potato, grains, flavors, colorants and spices.

In one arrangement, the animal food composition of the present invention is provided as a nutritionally complete animal food composition. A "nutritionally complete composition" is a composition that includes sufficient nutrients for maintenance of normal health of a healthy animal consuming the composition. In another arrangement, the composition of the present invention may be in the form of a treat, snack or supplement.

The present invention additionally provides a method of increasing the firmness of a food component, wherein the method comprises:
treating the food component with transglutaminase and at least one further agent selected from gluten and alginate ("the active agents"), in the absence of added heat or pressure.

The food component, treatment of the food component with the active agents, and the firming of the food component may be as defined herein.

In some embodiments, the present invention further provides an animal food composition comprising:
a protein source,
transglutaminase in an amount of 1 unit of enzyme activity/g to 4 units of enzyme activity/g of the food component,
   and
at least one agent selected from gluten in an amount of 0.05 wt% to 10 wt% by total weight of the food component, and alginate in an amount of 0.01 wt% to 3 wt% by total weight of the food component. The agent selected may be gluten, alginate, or gluten and alginate.

Transglutaminase may be obtained as defined herein. In one arrangement, the transglutaminase is present in an amount of 1 unit of enzyme activity/g to 4 units of enzyme activity/g of the food composition, or in an amount of 1.5,2, or 2.5 units of enzyme activity/g to 3, 3.5 or 4 units of enzyme activity/g of the food composition. Optionally, the transglutaminase is present in an amount of 1 unit of enzyme activity/ g to 3 units of enzyme activity/g of the food composition, or in an amount of 1.5 or 2 units of enzyme activity/g to 2.5 or 3 units of enzyme activity/g of the food composition. Still further optionally, the transglutaminase is present in an amount of 1 unit of enzyme activity/g to 2 units of enzyme activity/g of the food composition, or in an amount of 1.1, 1.2, 1.3, or 1.4 units of enzyme activity/g, to 1.5, 1.6, 1.7, 1.8 or 1.9 units of enzyme activity/g of the food composition,

The alginate may be in the form of sodium alginate, potassium alginate, ammonium alginate, calcium alginate and propylene glycol alginate as described herein. In one arrangement, the alginate is present in an amount of 0.05 wt% to 2 wt% by total weight of the food composition. Preferably, the alginate is present in an amount of 0.1 wt% to 1.5 wt% by total weight of the food composition, or in an amount of 0.5 wt% to 1 wt% by total weight of the food composition. In another arrangement, the alginate is present in an amount of from 0.1 wt%, or 0.2 wt%, or 0.3 wt% or 0.4 wt% or 0.5 wt% to 1 wt%, or 1.5 wt% or 2 wt% by total weight of the food composition.

Gluten may be obtained as described herein. In one arrangement, gluten is present in an amount of from 0.6 wt%, or 0.7 wt%, or 0.8 wt% or 0.9 wt% or 1 wt% to 1wt%, or 1.5 wt%, or 2 wt%, or 2.5 wt% or 3 wt%, or 5 wt%, or 7 wt% or 10 wt% by total weight of the food composition. In another arrangement, gluten is present in an amount of 1 wt% to 1.5 wt% by total weight of the food composition.

The composition may further comprise a protein source as defined herein.

In one arrangement, the food composition further comprises one or more food ingredients selected from grains, cereal flour, starch, fats, oils, vitamins, minerals, colorants, flavorants, amino acids, fiber, and inorganic additives. The food ingredients may be as defined herein.

The food composition preferably comprises a meat analogue as defined herein. In some embodiments, the food composition is cured and optionally, overwrapped. Still further optionally, the animal food composition is vacuum-sealed.

The term "animal" is as defined herein. Preferably, the animal is a companion animal as defined herein.

In some embodiments, the food composition is a moist composition.

The present invention further provides a use of transglutaminase and gluten to increase the firmness of a food component in the absence of added heat or pressure.

The present invention yet further provides a use of transglutaminase and alginate to increase the firmness of a food component in the absence of added heat or pressure.

The food component and the means by which the firmness of the food component is increased using the active agents, may be as defined herein.

The inventors have surprisingly found an unexpected synergistic effect between transglutaminase and gluten, and between transglutaminase and alginate. In particular, the combination of transglutaminase and gluten, or the combination of transglutaminase and alginate, provides a far superior effect on the structuring and firming of food components than any of the active agents alone. Furthermore, the synergistic combination allows firming to be achieved in the absence of added heat or pressure. The methods and uses of the present invention accordingly provide a new means by which the firmness of a food component may be increased, without requiring a heating step or added pressure. The invention is further illustrated in the following non-limiting Examples.

### Examples

### Example 1 - Initial Screening Test

In order to investigate the effects of transglutaminase on the firmness of food components, it was necessary to establish a protocol for preparing food chunks whose firmness could be measured.

600 grams of thawed low-fat and ash ground chicken, 200 grams of non-genetically modified rice flour, 87 grams water (at room temperature), and 31.1 grams of transglutaminase with 3.4 units of enzyme activity/g of food component was added to a 5-quart mixing bowl and mixed for 5 minutes to form a meat mixture.

Water was heated to 180°F (∼82°C) and 5 g boluses of the meat mixture were immersed in the heated water for three minutes to form chunks. The water was subsequently drained and the chunks were dried on a paper towel.

Separately, a sample of the meat mixture was covered and wrapped with Saran^{®}, and incubated at 4°C for 12 hours.

The addition of the meat mixture to the warm water resulted in the formation of small meat chunks. As the chunks cooled, they became slightly firmer.

However, incubation of the meat mixture at 4°C for 12 hours did not result in the formation of a solid product. It could be concluded that the added water rendered the meat mixture too soft to form a solid product. Thus, in subsequent experiments, water was not added to the initial meat mixture.

### Example 2 - Effects of transglutaminase (TG) on chunk firmness after heating

340 grams of thawed, ground beef-heart, 140 grams of non-genetically modified rice, and 8.71 grams of TI Transglutaminase (TG) flour with 1.8 units of enzyme activity/g of food component were added to a 5-quart mixing bowl and mixed for 5 minutes to form a meat mixture. The meat mixture was placed in small cylindrical containers to form uniformly sized pieces. (TG-treated and non-treated samples were tested in duplicate.) The containers were then placed in a water bath maintained at 180°F (∼82°C) for 5 minutes to form meat chunks. Subsequently, the chunks were removed, cooled in a cooling bath and refrigerated at 4°C for 12 hours.

The firmness of the refrigerated chunks was measured with a TA-XT2 penetrometer using a 4mm probe and 6mm penetration. The peak force observed during penetration was determined. The results are illustrated in Table 1. The higher the force measurement, the greater the firmness.

**Table 1**

| | Force without TG (g) | Force with TG (g) |
|---|---|---|
| Sample 1 | 5268.4 | 4624.0 |
| Sample 2 | 5600.5 | 5356.6 |
| Average | 5434.4 | 4990.3 |

As can be seen from Table 1, there was no significant difference in the firmness of the meat chunks that had been treated with TG prior to heating, and those that had not been treated with TG prior to heating. It could therefore be concluded that TG has no significant effect on the firmness of meat chunks if they are subsequently heated.

### Example 3 - Effects of transglutaminase (TG) on chunk firmness in the absence of heating

340 grams of thawed, ground beef-heart, 140 grams of non-genetically modified rice, and optionally, 8.71 grams of TI Transglutaminase (TG) flour with 1.8 units of enzyme activity/g of food component were added to a 5-quart mixing bowl and mixed for 5 minutes to form a meat mixture. The meat mixture was placed in small cylindrical containers to form uniformly sized pieces. (TG-treated and non-treated samples were tested in triplicate.) The containers were then refrigerated at 4°C for 18 hours. Following removal of the containers from the refrigerator, the containers were left at room temperature for 2 hours. The firmness of the chunks subsequently measured with a TA-XT2 penetrometer using a 4mm probe and 6mm penetration.

Table 2 indicates values for the mean force and mean peak force observed during penetration.

**Table 2**

| | Peak Force without TG(g) | Peak Force with TG (g) |
|---|---|---|
| | Mean Peak | Mean Peak |
| Sample 1 | 720.2 | 1388.1 |
| Sample 2 | 768.6 | 1312.3 |
| Sample 3 | 802.54 | 1482.5 |
| Average | 763.8 | 1394.3 |

As can be seen from Table 2, the test samples which had been treated with TG were significantly firmer than the untreated controls. Thus it could be concluded that TG significantly enhances the firmness of meat chunks without the requirement for any heating.

### Example 4 - Effects of transglutaminase (TG) on chunk firmness in the presence of viscosity modifiers

78% thawed, ground, low ash and low fat chicken and 28% non-genetically modified rice ('meat/rice flour blend'), were combined with the following ingredients in the following amounts, and mixed to form a homogenous blend in a Hobart mixer:
Test 1- 200 grams of meat/rice flour blend; 6 grams of wheat gluten
Test 2 -200 grams of meat/rice flour blend; 4 grams of 250 bloom gelatin dissolved in 4 grams of water
Test 3 - 200 grams of meat/rice flour blend; 1 gram of Sodium Alginate dissolved in 4 grams of water
Test 4 - 200 grams of meat/rice flour blend; 6 grams of soy protein isolate
Test 5 - 200 grams of meat/rice flour blend; 6 grams of dried granulated egg white
Test 6 - 100 grams of Test 1 mixture; 1.75 grams of Transglutaminase preparation with 1.7 units of enzyme activity/g of food component
Test 7 - 100 grams of Test 2 mixture; 1.75 grams of Transglutaminase preparation with 1.7 units of enzyme activity/g of food component
Test 8 - 100 grams of Test 3 mixture; 1.75 grams of Transglutaminase preparation with 1.7 units of enzyme activity/g of food component
Test 9 - 100 grams of Test 4 mixture; 1.75 grams of Transglutaminase preparation with 1.7 units of enzyme activity/g of food component
Test 10 - 100 grams of Test 5 mixture; 1.75 grams of Transglutaminase preparation with 1.7 units of enzyme activity/g of food component
Test 11 - 100 grams of meat/rice flour blend with no additives (No Additive Control)
Test 12 - 100 grams of meat/rice flour blend; 1.75 grams of Transglutaminase preparation with 1.7 units of enzyme activity/g of food component

The test mixtures were placed in small cylindrical containers to form uniformly sized pieces. (Test samples were prepared in duplicate.) The containers were then refrigerated at 4°C for 72 hours. Following removal of the containers from the refrigerator, the containers were left at room temperature for 3 hours. The firmness of the chunks subsequently measured with a TA-XT2 penetrometer using a 4mm probe and 6mm penetration, as described above in Examples 2 and 3.

The results are illustrated below in Table 3.

**Table 3**

| Test | Description | Mean Peak Force(g) (n = 2) | % increase in force for test with TG relative to corresponding test without TG |
|---|---|---|---|
| 1 | Meat/Flour Mixture + Gluten | 319.5 | N/A |
| 2 | Meat/Flour Mixture + Water/Gelatin | 443.6 | N/A |
| 3 | Meat/Flour Mixture + Water/Alginate | 1064.5 | N/A |
| 4 | Meat/Flour Mixture + Soy Protein Isolate | 370.0 | N/A |
| 5 | Meat/Flour Mixture + Dried Egg White | 185.0 | N/A |
| 6 | T1 + TG | 464.8 | 45.5 |
| 7 | T2 + TG | 520.4 | 17.2 |
| 8 | T3 + TG | 1564.7 | 47.0 |
| 9 | T4 + TG | 423.6 | 14.5 |
| 10 | T5 + TG | 213.5 | 15.4 |
| 11 | Meat/Flour Mixture | 199.5 (n = 1) | N/A |
| 12 | T11 + TG | 233.0 | 16.8 |

The addition of TG to a rice flour/ground chicken blend increased the firmness of the blend by 16.8%. The addition of gelatin, soy protein isolate, or dried egg white to the rice flour/chicken/TG blend did not appear to significantly affect the firmness of the mixture. However, the addition of wheat gluten or sodium alginate to the rice flour/chicken/TG blend increased the firmness by 45.5 and 47.0 percent, respectively. It may therefore be concluded that a synergic effect on increasing mixture firmness occurs when gluten or alginate are used in conjunction with TG.

Additionally, it can be seen that the mean peak force observed for the meat (chicken)/flour/TG mixture is considerable lower in Example 4, than the mean peak force observed for the meat (beef)/flour/TG mixture used in Examples 2 and 3. Thus it may be concluded that TG is more effective in increasing the firmness of meat chunks comprising beef than meat chunks comprising chicken.

### Example 5 - food composition

A preferred embodiment of the invention would contain the range of ingredients illustrated in Table 4.

**Table 4**

| Ingredient | Amount (wt%) by total weight of composition |
|---|---|
| Meat and Meat By-Products | 10-88 |
| Cereal Flour or Starches | 5-30 |
| Animal Proteins | 0-10 |
| Vegetable Proteins | 0-10 |
| Fiber | 0-14 |
| Lipid Ingredients | 0-6 |
| Vitamin Supplements | 0-3 |
| Mineral Supplements | 0-3 |
| Inorganic Additives | 0-3 |
| Water | 0-20 |
| Flavors | 0-3 |
| Colorants/Dyes | 0-3 |
| Crystalline Amino Acids | 0-3 |
| Transglutaminase Preparation | 0.075 to 1.75 (or 1 to 4 units/g) |
| Sodium or Potassium Alginate | 0.01 to 3 |

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. All percentages expressed herein are by weight of the composition on dry matter basis unless specifically stated otherwise.

As referred to herein, all enzyme activities are expressed in international units/g food (or food component), unless otherwise specified.

As used herein, the term "food" may refer not only to a food product which typically provides most, if not all, the nutrient value for an animal, but may also refer to items such as a snack, treat, and supplement.
The following description pages 21 to 29 contain a list of further general aspects of the invention.

### Further aspects of the invention

1. A method of preparing a moist animal food composition comprising a food component, wherein the method comprises:
   treating the food component with transglutaminase and at least one further agent selected from gluten and alginate,
   and firming the food component in the absence of added heat or pressure.
2. The method according to aspect 1, wherein the at least one further agent selected is gluten.
3. The method according to aspect 1 or aspect 2, wherein the food component is treated with gluten in an amount of 0.05 wt% to 10 wt% by total weight of the food component.
4. The method according to aspect 3, wherein the food component is treated with gluten in an amount of 0.5 wt% to 5 wt% by total weight of the food component.
5. The method according to aspect 1, wherein the at least one further agent selected is alginate.
6. The method according to aspect 1 or aspect 5, wherein the food component is treated with alginate in an amount of 0.01 wt% to 3 wt% by total weight of the food component.
7. The method according to aspect 6, wherein the food component is treated with alginate in an amount of 0.1 wt% to 1.5 wt% by total weight of the food component.
8. The method according to any of aspects 1 and 5 to 7, wherein the alginate comprises sodium alginate, potassium alginate, ammonium alginate, calcium alginate and propylene glycol alginate.
9. The method according to any preceding aspect, wherein the firming is conducted at a temperature of from 3°C to 23°C.
10. The method according to aspect 9, wherein the firming is conducted at a temperature of from 4°C to 10°C.
11. The method according to any preceding aspect, wherein the food component is treated with transglutaminase and the at least one further agent selected from gluten and alginate, for three to seventy-two hours.
12. The method according to aspect 11, wherein the food component is treated with transglutaminase and the at least one further agent selected from gluten and alginate, for six to eighteen hours.
13. The method according to any preceding aspect, wherein the food component comprises a protein source.
14. The method according to aspect 13, wherein the protein source comprises an animal protein source.
15. The method according to aspect 14, wherein the protein source comprises meat, a meat by-product or fish.
16. The method according to aspect 15, wherein the protein source comprises chicken, beef, pork or turkey.
17. The method according to aspect 16 wherein the protein source comprises beef.
18. The method according to aspect 14, wherein the food component further comprises a vegetable protein source.
19. The method according to any preceding aspect, wherein the food component is combined with one or more food ingredients.
20. The method according to aspect 19, wherein the food component is combined with one or more food ingredients before treating and/or firming.
21. The method according to aspect 19 or aspect 20, wherein the food component is combined with one or more food ingredients selected from grains, cereal flour, starch, fats, oils, vitamins, minerals, colorants, flavorants, amino acids, fiber, and inorganic additives.
22. The method according to any preceding aspect, wherein the food component further comprises one or more food ingredients selected from grains, cereal flour, starch, fats, oils, vitamins, minerals, colorants, flavorants, amino acids, fiber, and inorganic additives.
23. The method according to any preceding aspect, wherein food component is treated with transglutaminase in an amount of 1 unit of enzyme activity/g to 4 units of enzyme activity/g of the food component.
24. The method according to aspect 23, wherein the food component is treated with transglutaminase in an amount of 1 unit of enzyme activity/g to 3 units of enzyme activity/g of the food component.
25. The method according to aspect 24, wherein the food component is treated with transglutaminase in an amount of 1 unit of enzyme activity/g to 2 units of enzyme activity/g of the food component.
26. The method according to any preceding aspect, wherein the animal food composition is extruded and optionally, cut.
27. The method according to any preceding aspect, wherein the animal food composition is cured, and optionally, overwrapped.
28. The method according to any preceding aspect, wherein the animal food composition is vacuum-sealed.
29. The method according to any preceding aspect, wherein the animal is a companion animal.
30. The method according to any preceding aspect, wherein the firming is conducted at atmospheric pressure.
31. The method according to any preceding aspect, wherein the firming is conducted in the absence of exogenous moisture.
32. The method according to any preceding aspect, wherein the treating and firming are conducted simultaneously.
33. A method of increasing the firmness of a food component comprising:
   treating the food component with transglutaminase and at least one further agent selected from gluten and alginate, in the absence of added heat or pressure.
34. The method according to aspect 33, wherein the at least one further agent selected is gluten.
35. The method according to aspect 33 or aspect 34, wherein the food component is treated with gluten in an amount of 0.05 wt% to 10 wt% by total weight of the food component.
36. The method according to aspect 35, wherein the food component is treated with gluten in an amount of 0.5 wt% to 5 wt% by total weight of the food component.
37. The method according to aspect 33, wherein the at least one further agent selected is alginate.
38. The method according to aspect 33 or aspect 37, wherein the food component is treated with alginate in an amount of 0.01 wt% to 3 wt% by total weight of the food component.
39. The method according to aspect 37, wherein the food component is treated with alginate in an amount of 0.1 wt% to 1.5 wt% by total weight of the food component.
40. The method according to any of aspects 33, and 37 to 39, wherein the alginate comprises sodium alginate, potassium alginate, ammonium alginate, calcium alginate and propylene glycol alginate.
41. The method according to any of aspects 33 to 40, wherein the food component is treated with transglutaminase and the at least one further agent selected from gluten and alginate at a temperature of from 3°C to 23°C.
42. The method according to aspect 41, wherein the food component is treated with transglutaminase and the at least one further agent selected from gluten and alginate at a temperature of from 4°C to 10°C.
43. The method according to any of aspects 33 to 42, wherein the food component is treated with transglutaminase and the at least one further agent selected from gluten and alginate, for three to seventy-two hours.
44. The method according to aspect 43, wherein the food component is treated with transglutaminase and the at least one further agent selected from gluten and alginate, for six to eighteen hours.
45. The method according to any of aspects 33 to 44, wherein the food component comprises a protein source.
46. The method according to aspect 45, wherein the protein source is an animal protein source.
47. The method according to aspect 46, wherein the protein source comprises meat, a meat by-product or fish.
48. The method according to aspect 47, wherein the protein source comprises chicken, beef, pork or turkey.
49. The method according to aspect 48, wherein the protein source comprises beef.
50. The method according to any of aspects 45 to 49, wherein the food component further comprises a vegetable protein source.
51. The method according to any of aspects 33 to 50, wherein the food component is combined with one or more food ingredients.
52. The method according to aspect 51, wherein the food component is combined with one or more food ingredients before treating.
53. The method according to aspect 51 or aspect 52, wherein the food component is combined with one or more food ingredients selected from grains, cereal flour, starch, fats, oils, vitamins, minerals, colorants, flavorants, amino acids, fiber, and inorganic additives.
54. The method according to any of aspects 33 to 53, wherein the food component further comprises one or more food ingredients are selected from grains, cereal flour, starch, fats, oils, vitamins, minerals, colorants, flavorants, amino acids, fiber, and inorganic additives.
55. The method according to any of aspects 33 to 54, wherein food component is treated with transglutaminase in an amount of 1 unit of enzyme activity/g to 4 units of enzyme activity/g of the food component.
56. The method according to aspect 55, wherein the food component is treated with transglutaminase in an amount of 1 unit of enzyme activity/g to 3 units of enzyme activity/g of the food component.
57. The method according to aspect 56, wherein the food component is treated with transglutaminase in an amount of 1 unit of enzyme activity/g to 2 units of enzyme activity/g of the food component.
58. The method according to any of aspects 33 to 57, wherein the treatment is conducted at atmospheric pressure.
59. The method according to any of aspects 33 to 58, wherein the treatment is conducted in the absence of exogenous moisture.
60. An animal food composition comprising:
   a protein source,
   transglutaminase in an amount of 1 unit of enzyme activity/g to 4 units of enzyme activity/g of the food component,
      and
   at least one agent selected from gluten in an amount of 0.05 wt% to 10 wt% by total weight of the food component, and alginate in an amount of 0.01 wt% to 3 wt% by total weight of the food component.
61. The food composition according to aspect 60, wherein the food composition comprises transglutaminase in an amount of 1 unit of enzyme activity/g to 4 units of enzyme activity/g of the food component.
62. The food composition according to aspect 60 or aspect 61, wherein the food composition comprises transglutaminase in an amount of 1 unit of enzyme activity/g to 3 units of enzyme activity/g of the food component.
63. The food composition according to any of aspects 60 to 62, wherein the food composition comprises gluten in an amount of 0.5 wt% to 5 wt% by total weight of the food component.
64. The food composition according to any of aspects 60 to 63, wherein the food composition comprises alginate in an amount of 0.1 wt% to 1.5 wt% by total weight of the food component.
65. The food composition according to any of aspects 60 to 64, wherein the protein source comprises an animal protein source.
66. The food composition according to aspect 65, wherein the protein source comprises meat, a meat by-product or fish.
67. The food composition according to aspect 66, wherein the protein source comprises chicken, beef, pork or turkey.
68. The food composition according to aspect 67, wherein the protein source comprises beef.
69. The food composition according to any of aspects 65 to 68, wherein the food composition further comprises a vegetable protein source.
70. The food component according to any of aspects 60 to 69, wherein the food composition further comprises one or more food ingredients selected from grains, cereal flour, starch, fats, oils, vitamins, minerals, colorants, flavorants, amino acids, fiber, and inorganic additives.
71. The food composition according to any of aspects 60 to 70, wherein the alginate is sodium alginate, potassium alginate, ammonium alginate, calcium alginate and propylene glycol alginate.
72. The food composition according to any of aspects 60 to 71, wherein the animal is a companion animal.
73. The food composition according to any of aspects 60 to 72, wherein the food composition comprises a meat analogue.
74. The food composition according to any of aspects 60 to 73, wherein the food composition is cured and optionally, overwrapped.
75. The food composition according to any of aspects 60 to 74 wherein the animal food composition is vacuum-sealed.
76. Use of transglutaminase and gluten to increase the firmness of a food component in the absence of added heat or pressure.
77. Use of transglutaminase and alginate to increase the firmness of a food component in the absence of added heat or pressure.

## Claims

1. A method of preparing a moist animal food composition comprising a food component, wherein the method comprises:
treating the food component with transglutaminase and alginate; and
firming the food component,
wherein the food component is treated with the transglutaminase and the alginate for three to seventy-two hours,
wherein the treatment and firming are conducted in the absence of added heat or pressure, and
wherein the firming is conducted in the absence of exogenous moisture.

2. The method according to claim 1, wherein the alginate is present in an amount of from 0.01 wt% to 3 wt%, optionally, 0.1 wt% to 1.5 wt%, by total weight of the food component.

3. The method according to any preceding claim, wherein the alginate comprises at least one of sodium alginate, potassium alginate, ammonium alginate, calcium alginate, and propylene glycol alginate.

4. The method according to any preceding claim, wherein the firming is conducted at a temperature of from 3°C to 23°C, optionally 4°C to 10°C.

5. The method according to any preceding claim, wherein the food component is treated with the transglutaminase and the alginate, for six to eighteen hours.

6. The method according to any preceding claim, wherein the food component comprises a protein source, optionally, the protein source comprises an animal protein source, optionally, the protein source comprises meat, a meat by-product, or fish, further optionally, the protein source comprises chicken, beef, pork, or turkey.

7. The method according to any preceding claim, wherein the food component further comprises a vegetable protein source.

8. The method according to any preceding claim, wherein the food component is combined with one or more food ingredients before treating and/or firming, optionally, the one or more food ingredients is selected from grains, cereal flour, starch, fats, oils, vitamins, minerals, colorants, flavorants, amino acids, fiber, and inorganic additives.

9. The method according to any preceding claim, wherein food component is treated with transglutaminase in an amount of 1 unit of enzyme activity/g to 4 units of enzyme activity/g of the food component, optionally, in an amount of 1 unit of enzyme activity/g to 3 units of enzyme activity/g of the food component, further optionally, in an amount of 1 unit of enzyme activity/g to 2 units of enzyme activity/g of the food component.

10. The method according to any preceding claim, wherein the treating and firming are conducted simultaneously.

11. A method of increasing the firmness of a food component comprising treating the food component with transglutaminase and alginate, wherein the food component is treated in the absence of added heat or pressure, in the absence of exogenous moisture, and over a period of three to seventy-two hours.

12. An animal food composition, comprising:
a food component;
a protein source;
transglutaminase in an amount of 1 unit of enzyme activity/g to 4 units of enzyme activity/g of the food component; and
alginate in an amount of 0.01 wt% to 3 wt% by total weight of the food component.
